# EUROPEAN PATENT APPLICATION

(11) **EP 0 905 512 A1**
(43) Date of publication of application: **31.03.1999**
(21) Application number: 97402239.4
(22) Date of filing: 25.09.1997
(51) Int. Cl.: G01N 31/00, G01N 33/53, G01N 33/566, C07D 401/14, C07D 295/205, C07D 413/12, C07D 471/10, C07D 405/14, C07D 401/04, C07D 207/14, C07D 263/58, C07C 271/16

(54) **Method of identification of leads or active compounds**

(71) Applicant: Cerep, 75007 Paris (FR)
(72) Inventor: Jean, Thierry, 86000 Poitiers (FR); Chapelain, Béatrice, 86000 Poitiers (FR)
(74) Representative: Becker, Philippe

(57) **Abstract**

The invention relates to methods of selection of leads or active compounds from libraries. More specifically, the invention provides methods of selection of leads or compounds which are active and selective towards a desired target. In particular, the invention provides a screening method for the identification of leads from a library of compounds, said method comprising the steps of :
(a) screening a library of compounds for activity against a desired target, to obtain potent hits;
(b) profiling the potent hits of step (a) to obtain potent and selective leads.

The instant invention can be used for instance in drug discovery processes.

## Description

The invention relates to methods of selection of leads or active compounds from libraries. More specifically, the invention provides methods of selection of leads or compounds which are active and selective towards a desired target. The instant invention can be used for instance in drug discovery processes.

The current methodologies in drug discovery involves the screening of large quantities of compounds of natural origin or obtained through combinatorial chemistry methods.

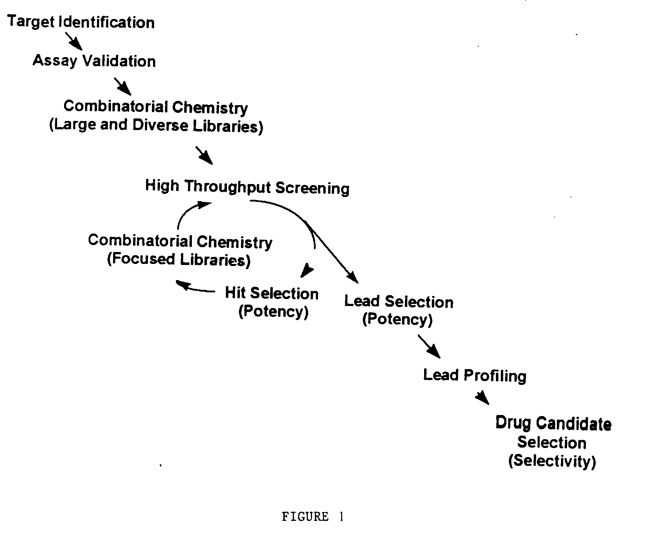

Libraries of several tens or hundreds of thousand of compounds are commonly tested in a single program of a drug discovery (figure 1). The selected compounds, namely hits, may be very numerous. The average hit rate is considered to be 0.1 %. Thus the screening of a 100,000 compounds library is likely to generate 100 hits. From the hit to the drug candidate is a long process involving multiple cycles of synthesis and screening of focused chemical libraries. This process of lead optimization is very time consuming and requires large budgets.

Indeed, hits to be followed up are commonly selected on the basis of the potency of their activity on the target screened. Each lead optimization cycle should allow to generate more active compounds. When a few hits exhibit the expected potency, usually after several optimization cycle, they become leads which are then tested for their selectivity against a large number of biological targets ("lead profiling"). The drug candidates are retained on the basis of their affinity for the chosen target and their specificity for this target (i.e. they should not be active on targets unrelated to the pathology object of the program). It is not rare that the selected leads prove to be unspecific, being thus likely to cause undesired effects. It is then necessary to start over the process from a less potent hit.

The present invention relates to improved methods of identification of leads or active compounds from libraries. The invention also relates to a high throughput profiling method, which allows the screening of selective compounds from large and diverse compositions. The invention also provides novel focused libraries comprising a plurality of compounds with structural diversity, but common functional properties.

The methods of the instant invention involve more specifically the determination of hit specificity ("profiling") at a very early stage in the drug discovery process. These new methods allow the identification of leads with improved properties (i.e. combining the criteria of potency and selectivity towards a given target) which can, optionally, be used in lead optimization processes to identify drug candidates with a higher predictability and lower costs.

It is therefore an object of the present invention to provide a method for the identification of leads from a library of compounds, said method comprising the steps of:
(a) screening a library of compounds for activity against a desired target, to obtain potent hits;
(b) profiling the potent hits of step (a) to obtain potent and selective leads.

As explained above, previous methods of drug discovery or lead selection involve a screening of a library of compounds for activity against a desired target, and the hits thereby obtained are then further processed through a lead optimization cycle to produce the leads. However, these steps are only based on the screening of libraries or focused libraries for a desired target, i.e. on the selection of compounds having the capacity to interact with a given target. None of the previous methods, however, involves a profiling step according to the instant invention. The high throughput screening method of the instant invention provides such a profiling step of the library so that the leads obtained combine both criteria of potency (capacity to interact with a given target) and selectivity (low or lack of interaction with other targets).

It is another object of the present invention to provide a method for the identification of active compounds from a library of compounds, said method comprising:
(i) a high throughput screening comprising the steps of:
   (a) screening a library of compounds for activity against a desired target, to obtain potent hits;
   (b) profiling the potent hits of step (a) to obtain potent and selective leads;
   (c) preparing focused library(ies) of compounds structurally related to the leads of step (b);
(ii) optionally repeating, one or several times, step (i) using the focused library(ies) of step (c) to generate further focused libraries, and,
(iii) screening the focused libraries of step (i) or (ii) for said target, to obtain active compounds.

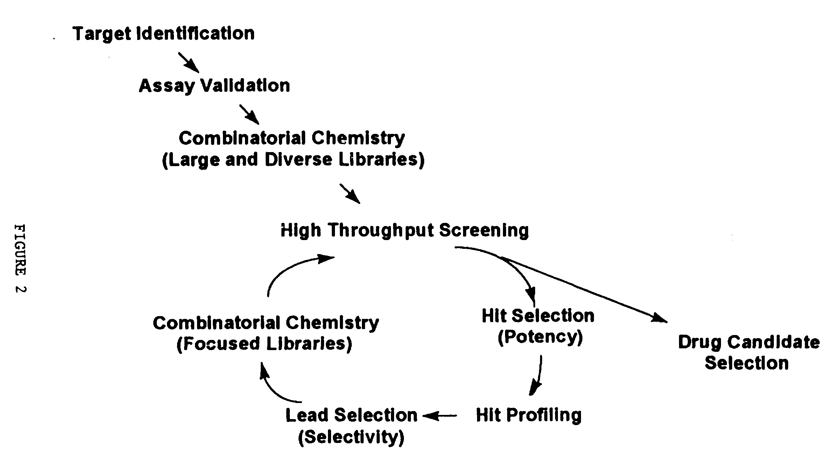

This method is illustrated in Figure 2 and comprises both the sequential steps of the lead identification method as disclosed above and the lead optimization steps, which produce active compounds.

For sake of clarity, the term "hit" designates, within the meaning of the instant invention, any compound selected after one screening step of a primary library. A hit is therefore a compound obtained after the first level of selection, which thus has at least the minimum potency vis-a-vis a desired target. A "lead" is an optimized hit, i.e. a hit whose activity has been confirmed, more preferably using a different selection test. Leads are therefore compounds obtained after at least 2 screening steps. Leads represent starting material for further optimization steps comprising the design of focus libraries, selection of active compounds, chemical modification thereof, functional testing, etc., which will produce drug candidate(s).

In the methods of the instant invention, a library is screened for activity against a chosen target. In general terms, the target can be any molecule such as for instance a receptor, a protein, or a portion of a receptor, or an antibody, a ligand, or an infectious particle such as a virus, a bacteria, a fungus or a nucleic acid, etc..., either in the pharmaceutical or in the agrochemical domain. More preferably, the target is a molecule involved in a biological or pathological pathway. Particular types of targets are for instance receptors, such as those recited in Table 1.

Step (a) of the claimed methods comprises the screening of the above libraries for activity against a given target. The screening for activity against a given target relates, in a general way, to the selection of any compounds of the library having the capacity to interact with the said target. Interaction can be physical or functional. More preferably, the screening of step (a) comprises the selection of compounds capable of modifying the activity of an enzyme, regulating the expression of a gene or binding to said desired target. As an example, the binding can be assayed according to any method known in the art, such as immunoenzymatic methods, Proximity Scintillation Assay or by radioassay (ELISA, IRA, etc.). In a preferred embodiment, the capacity of a compound to bind to a given target is measured by the capacity of said compound to inhibit the binding, to said target, of a reference, labelled molecule. As an example, the capacity of a compound to bind to a given target receptor can be measured by the capacity of said compound to inhibit the binding, to said target receptor, of a labelled ligand of said receptor. In this particular case, the screening therefore comprises, for each compound, the determination of the percentage of inhibition of the binding to the target of a labelled ligand.

In order for this screening method to be more accurate, it is preferred that the capacity of the compounds to bind to the target be confirmed. Confirmation may be obtained for instance by simply repeating the above screening method, but using the compound in purified form rather than contained in the whole library.

Depending on the size of the starting library, the number of confirmed hits may vary between 5 to 500, usually between 5 to 100.

Furthermore, in a preferred embodiment, the screening of step (a) further comprises measuring the affinity of the compounds capable of binding to said desired target, in order to select, as potent hits, the compounds having the best affinity. In order to determine the affinity, the confirmed hits are usually re-synthesized and purified. The affinity is then determined by methods known in the art, such as by "Scatchard" analysis as disclosed in the examples. Also, during this step, compounds can further be discarded because of their chemical structure.

Compounds screened according to step (a), generally between 5 to 50, are then subjected to step (b). The profiling step (b) comprises in general terms assaying the potent hits on various targets and selecting the compounds which are specific for the desired target. This step therefore allows to retain leads which exhibit both the expected potency for the target and a good selectivity profile. Step (b) usually comprises determining the capacity of the selected hits to interact with, preferably to bind to various targets.

In a particular embodiment of this invention, step (b) comprises assaying the potent hits on more than 25 targets. In a more particular embodiment, said profiling step (b) comprises assaying the potent hits on more than 50 targets. In a most preferred embodiment, the profiling step (b) comprises assaying the potent hits on more than 60 targets. Illustrative examples of targets than can be assayed in step (b) are listed in Table I below. The capacity of the compounds to interact with or bind to these targets can be determined following the same methodology as described in step (a). The compounds retained after this step (b) are those which bind to the desired target and which do not bind to any other target or bind only to a few of them or poorly to other targets. The selectivity requirements can vary from one target to another and can be adapted by the skilled artisan. This step (b) is very important in that it determines the level of selectivity which is sought is the drug discovery process. In this respect, to facilitate this profiling step (b), Applicants have developed a concept of High Throughpout Profiling ("HTP") which allows to quickly test in vitro numerous compounds (for instance in the range of one to two hundreds) in a large number of targets. This HTP involves a robotic system which is able to run up to 20 protocols (one protocol per target) per day. This method is disclosed more in details in the examples.

The leads thereby obtained can be used in step (c) as templates to design and synthesize focused libraries of compounds structurally related thereto. These focused libraries can be produced according to the techniques disclosed below and known in the art, and may contain several thousands of compounds. The term "structurally related" indicates that these libraries are designed so that their compounds contain structures or motifs which are also present in the leads. For instance, these libraries may contain specific monomers or blocks identified in the leads, as disclosed in the examples.

From these focused libraries, steps (a) to (c) can be repeated to obtain further focused libraries (step (ii)). Depending on the selectivity or potency which are sought, this cycle may be repeated from 0 to 10 times, preferably from 1 to 5 times.

These libraries are finally screened for the given target (step (iii)), to obtain active compounds. The screening of step (iii) can be carried out according to methods known in the art. Preferably, the screening of step (iii) is performed in a similar way as the screening and profinling of steps (a) and (b) above. This screening may also contain additional steps such as a genotoxicity assay or a functional assay as described in the examples.

The methods disclosed above can be applied to various libraries of compounds, including libraries of compounds of natural origin or obtained through well-known combinatorial chemistry methods. These combinatorial chemistry methods involve the synthesis of random or focused series of compounds starting from monomers or blocks, using for instance solid support- or liquid synthesis, parallel synthesis, microbeads, divide couple recombine or any other method known in the art (N.K. Terret, M. Gardner, D.W. Gordon, R.J. Kobylecki, J. Steele. "Combinatorial synthesis. The design of compound libraries and their application to drug discovery". Tetrahedron, Vol. 51, N° 30, (1995) pp 8135-8173). The compounds can therefore be nucleic acids, peptides, other chemicals or a combination thereof. These libraries may for instance comprise between 5 000 and 500 000 compounds, for instance between 10 000 and 20 000 compounds.

The methods of the invention can be used efficiently to identify various types of active compounds such as drug candidates, receptor ligands. The advantages of these methods are the possibility to produce leads which are both potent and selective for a target, thereby avoiding the follow-up of poor leads.

In this respect, the invention also relates to a method of High Throughput Profiling comprising the screening of a large number of compounds for their capacity to interact with one given target amongts a large number of targets. More preferably, the number of compounds tested is comprised between 5 and 500, more specifically between 5 and 100, such as for instance between 5 and 50. The number of targets assayed in preferably above 25, more prefarably above 50. It is particularly preferred to use a number of target above 60, such as for instance 62 or 73. An illustrative list of such targets is provided in Table I. The screening of the compounds is usually performed by incubating each compound with each target, followed by the determination of an interaction (e.g. a binding). Such screening may be carried out for instance in microtitration plates, so as to perform as many screen as possible in a limited period of time. The invention disclosed in this respect a robotic system allowing this screening to be performed rapidly, such robotic system providing for an automated distribution of the compounds in the plates followed by a determination of the capacity of the compound to inhibit binding of ligand to the target. To carry out this method, the plates usually comprise the target coated to their surface, as well as a labelled ligand. As shown in the Examples, this method allows the determination, in a relatively short period of time, of various compounds on numerous targets (73).

The present invention also provides improved libraries of compounds. In particular, the invention provides focused libraries comprising a plurality of compounds, wherein said compounds have structural diversity and derive from compounds having the capacity to bind selectively to a common desired target. While prior art focused libraries comprise essentially compounds deriving from hits having the capacity to bind to a target, the invention now provides more defined libraries, which comprise compounds deriving from hits which have not only the capacity to bind to a target, but which are also specific for said target. These libraries are thus more focused than the previous ones and can be used as a source of compounds in a drug discovery program. The term "deriving" in this context means obtained by combinatorial chemistry methods based on the structure of such hits, according to methods known in the art.

The instant invention will be disclosed in more details in the following examples, which are only illustrative and in no way limit the scope of the instant invention.

### Legends to the Figures

- Figure 1:: Classical drug discovery process
- Figure 2:: Drug discovery process of the invention
- Figure 3 :: Determination of the IC50 of selected hits for the rat mu receptor. Refence compound: DAGO.
- Figure 4:: Structure of 12 leads.
- Figure 5:: Determination of the affinity of the selected hits for the human mu receptor.
- Figure 6 :: HTP data for selected hits. The compounds selected from the affinity study have been tested on multiple targets. The identity of the targets is as described in Table I. The effect is indicated by the intensity of the grey in each case, white being inactive and black being the maximal effect. At the bottom of the figure, data from Table 2 are reported, namely affinity values of the compounds for the opioid receptors.
- Figure 7 :: Functional assay : Determination of the inhibition of guinea-pig ileum contraction by the selected leads.

### Examples

### Example 1 : hit selection for the opioid mu receptor

A library of 10,000 compounds has been designed, synthesized and screened in a model of opioid mu receptors from rat origin using a classical binding assay system. More specifically, compounds were tested in solution, at a 10⁻⁵M concentration, in the presence of [³H] DAMGO as radioligand on rat cortex homogenate. Specific binding was determined under competition with naloxone by liquid scintillation.

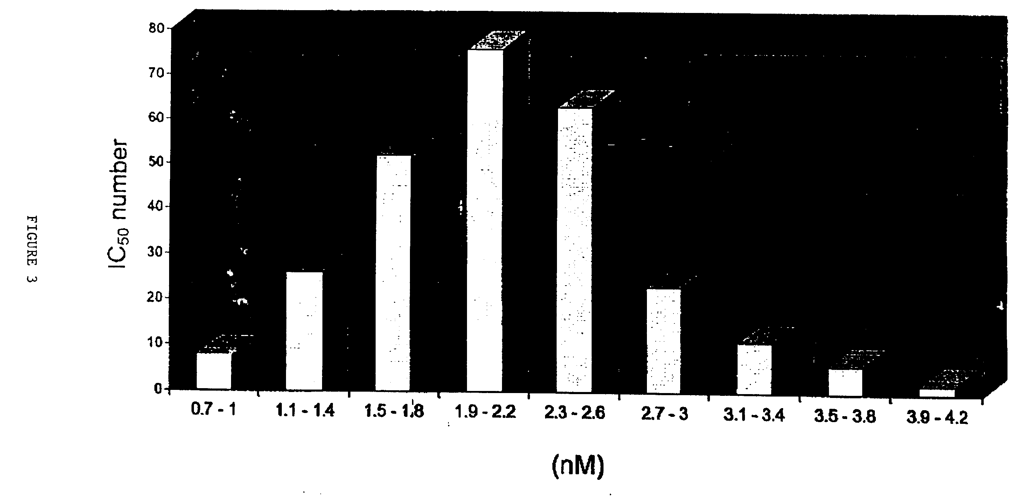

This primary screening resulted in numerous hits (Figure 3): 180 compounds inhibited more than 50% the binding of the radioligand specific to the mu receptor. 111 inhibited more than 60% of said binding and 42 more than 70%. In order to obtain active compounds with a high potency, these latter 42 compounds have been used for the further steps.

The chemical structure of these 42 compounds have been analysed.

Among these 42 compounds, 24 were discarded because of their chemical structure (i.e. incompatibility with industrial applications) and 18 were retained for further analysis.

One of these compounds (CER680827) was not confirmed to be active in the rat mu assay. The 17 remaining compounds were re-synthesized according to methods known in the art, purified and their affinity was measured in human mu, delta and kappa receptor binding assays.

As shown in Table II, five compounds proved to be inactive (CER680435, CER728422, CER728428, CER829741, CER838941), either because the effect observed in the primary screening was due to a side product (primary screening is performed with unpurified compounds) or because of a species selectivity of these compounds (primary screening was performed using the rat receptor and affinity was determined using the human receptor).

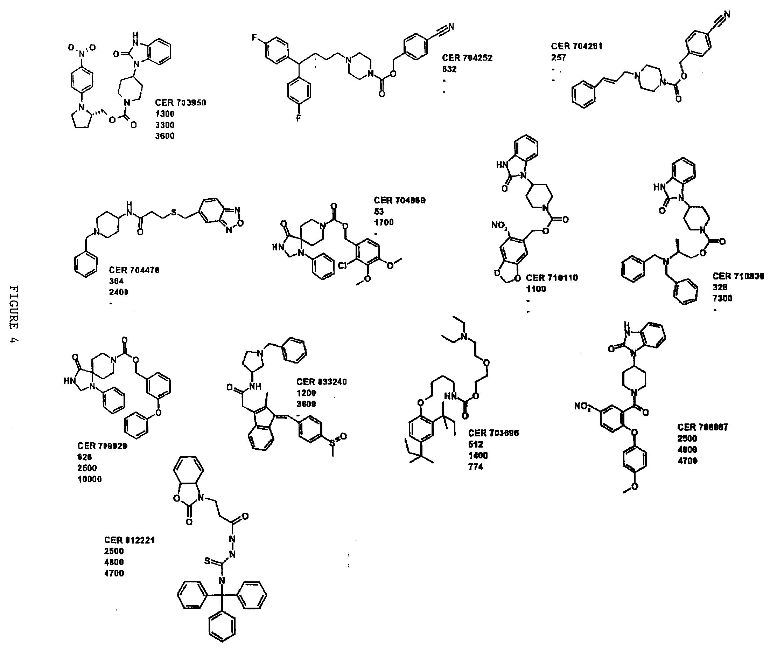

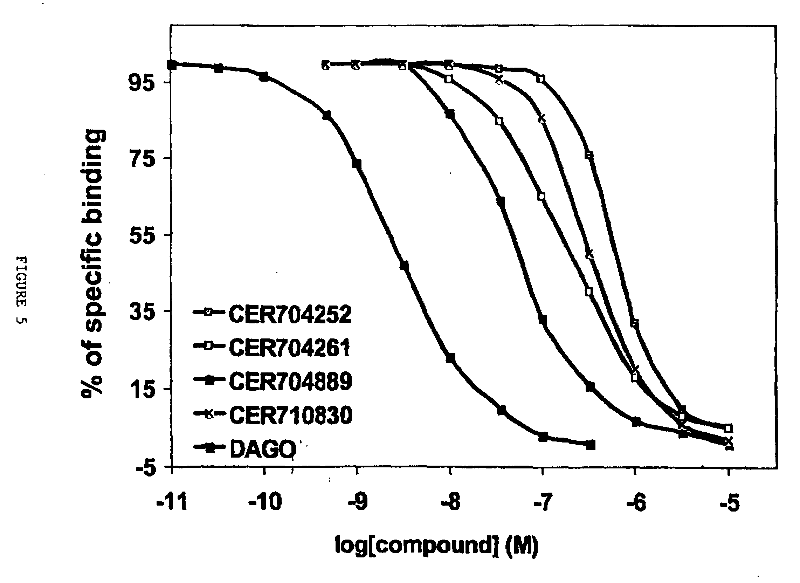

However, 12 hits exhibited the expected affinity for the mu receptor and only one of them (CER798967) was found to be more active on the delta receptor than on the mu receptor. The structure of these 12 compounds is represented on Figure 4. Data are summarized in Figure 5 and Table II.

These 12 compounds have then been tested in the HTP system, i.e. their selectivity profile has been determined using 62 receptors as described in Table I. For that purpose, 10 µM of each compound is incubated in microtitration plates previously coated with the targets shown in Table I, in the presence of a reference labelled ligand specific for each of said target. Each compound is tested in duplicate.

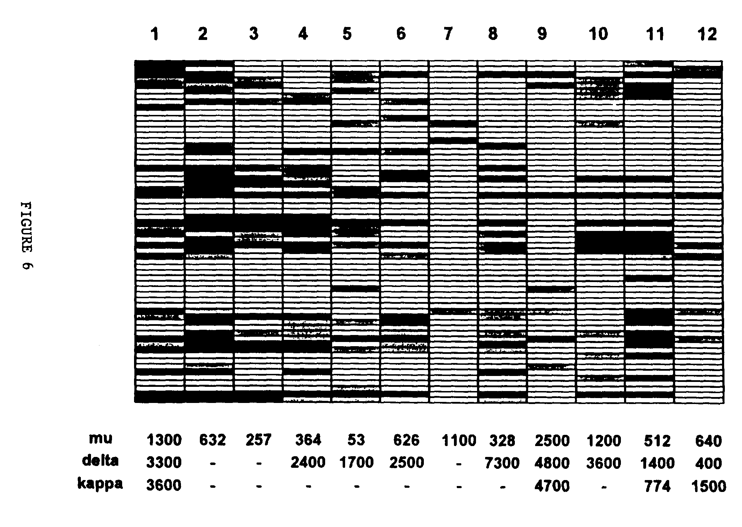

The results obtained are summarized in Figure 6. The HTP gave precious information since it allowed to show that some of the compounds which could have been selected on the basis of their relative affinity for opioid receptors proved to be active on many unrelated targets. These compounds have thus be discarded because of their unspecificity.

Combining the criteria of potency (affinity of the hits to the mu and other opioid receptors) and selectivity allowed to select 4 compounds (leads) as candidates for the follow-up of the lead optimization process. These compounds are CER704252, CER704261, CER704889 and CER710830.

In addition, in order to confirm the functionality of the lead compounds obtained according to the instant method, each of these compounds was tested for its capacity to inhibit guinea-pig ileum contraction as follows:

### Experimental procedures:

Ileum segments are obtained from male Dunkin Hartley guinea-pigs weighing 300-350 g. The tissues are suspended between two stainless-steel hooks in organ baths containing an oxygenated and pre-warmed physiological salt solution where they are connected to force-displacement transducers for isometric tension recording. Twitch contractions are elicited by field electrical stimulation (single pulses, 1 ms duration, maximal intensity, 0.1 Hz) delivered through two platinum electrodes by a multichannel constant current stimulator. The tissues are stretched to an optimal resting tension and then allowed to equilibrate for at least 30 min during which time they are washed repeatedly. Drugs are added after the twitch contraction amplitude is reproducible.

### Test for agonist activity:

The tissues are initially exposed to an effective concentration of the reference agonist DAMGO to verify tissue responsiveness. Following washout and another equilibration period, the tissues are exposed to several increasing concentrations of the test compounds or the same agonist. The different concentrations are added cumulatively and cach remains in contact with the tissues until a stable response is obtained. When an agonist-like responses (inhibition of twitch contractions) is obtained, the reference antagonist naloxone is added after the action of the highest concentration of the test compounds to check the involvement of µ receptors in this response.

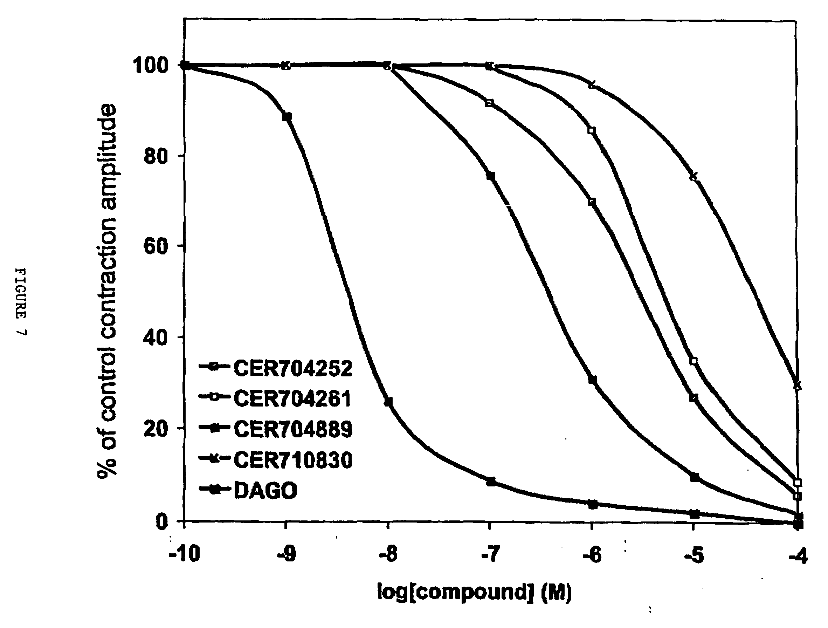

The results presented on Figure 7 confirm that the selected leads have all the capacity to inhibit guinea-pig ileum contraction which further demonstrates the reliability of the method of the invention.

All together, this first cycle of lead optimization has taken 6 weeks (synthesis, primary screening, confirmation, affinity to opioid receptors and HTP) and could be reduced to 3 weeks, mainly by decreasing the turnaround time of HTP. This will make the lead discovery both rapid and secure, in the sense that it will avoid the follow-up of poor lead candidates.

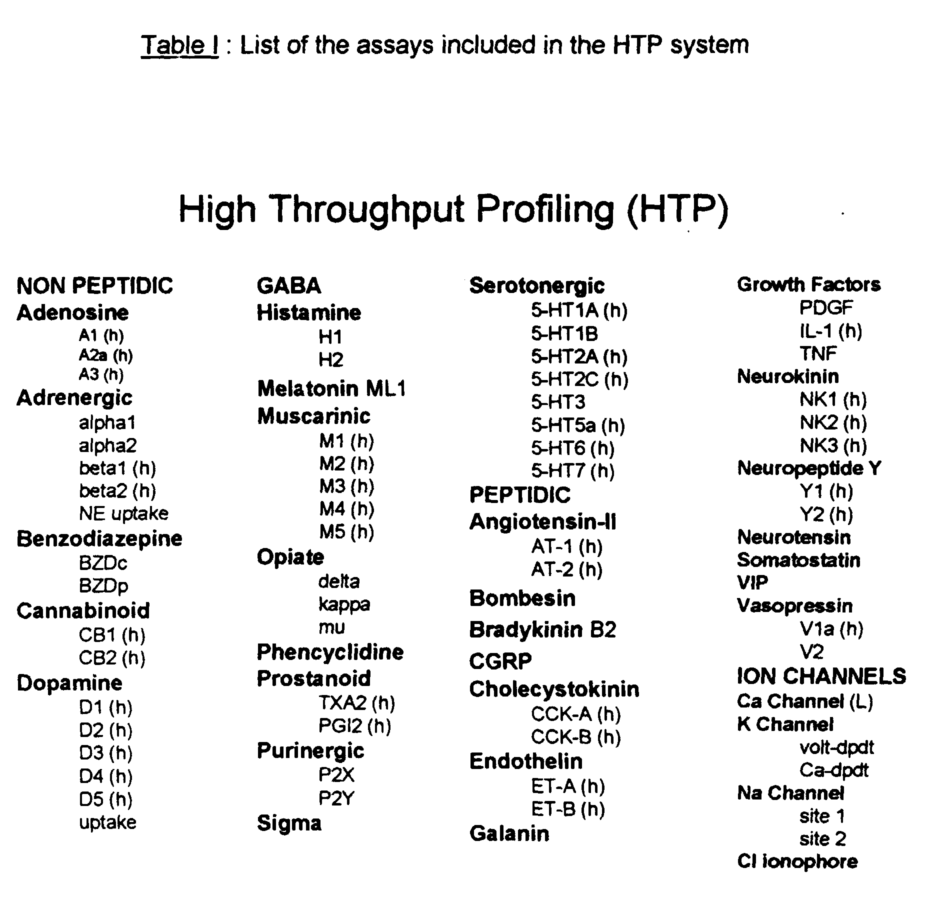

**Table II**

| Affinity of hits on the opioid receptors | | | | | | | |
|---|---|---|---|---|---|---|---|
| **Compound** | **mu** | **delta** | **kappa** | **Compound** | **mu** | **delta** | **kappa** |
| **CER680435** | **-** | **-** | **-** | **CER710830** | **328** | **7300** | **-** |
| **CER680827** | **-** | **-** | **-** | **CER728422** | **-** | **-** | **-** |
| **CER703950** | **1300** | **3300** | **3600** | **CER728428** | **-** | **-** | **-** |
| **CER704252** | **632** | **-** | **-** | **CER812221** | **2500** | **4800** | **4700** |
| **CER704261** | **257** | **-** | **-** | **CER829741** | **-** | **8400** | **-** |
| **CER704478** | **364** | **2400** | **-** | **CER838941** | **-** | **-** | **-** |
| **CER704889** | **53** | **1700** | **-** | **CER833240** | **1200** | **3600** | **-** |
| **CER709929** | **626** | **2500** | **10000** | **CER703696** | **512** | **1400** | **774** |
| **CER710110** | **1100** | **-** | **-** | **CER798967** | **640** | **400** | **1500** |
| *Affinities of compounds are given in nM. The sign - indicates the absence of* e*ffects in the concentration range tested (0.1 to 10,000 nM)* | | | | | | | |

## Claims

1. A high throughput screening method for the identification of leads from a library of compounds, said method comprising the steps of:
(a) screening a library of compounds for activity against a desired target, to obtain potent hits;
(b) profiling the potent hits of step (a) to obtain potent and selective leads.

2. A method of identification of active compounds from a library of compounds, said method comprising :
(i) a high throughput screening comprising the steps of :
(a) screening a library of compounds for activity against a desired target, to obtain potent hits;
(b) profiling the potent hits of step (a) to obtain potent and selective leads;
(c) preparing focused library(ies) of compounds structurally related to the leads of step (b);
(ii) optionally repeating, one or several times, step (i) using the focused library(ies) of step (c) to generate further focused libraries, and,
(iii) screening the focused libraries of step (i) or (ii) for activity against said target, to obtain active compounds.

3. Method of claim 1 or 2 wherein said screening of step (a) comprises the selection of compounds capable of interacting to a desired target.

4. Method of claim 2 wherein said screening of step (a) further comprises measuring the affinity of the compounds capable of binding to said desired target, in order to select, as potent hits, the compounds having the best affinity.

5. Method of claim 1 or 2 wherein said profiling step (b) comprises assaying the potent hits on various targets and selecting the compounds which are specific for the desired target.

6. Method of claim 4 wherein said profiling step (b) comprises assaying the potent hits on more than 25 targets.

7. Method of claim 5 wherein said profiling step (b) comprises assaying the potent hits on more than 50 targets.

8. Method of claim 6 wherein said profiling step (b) comprises assaying the potent hits on more than 70 targets.

9. Method of claim 1, wherein, in step (b), between 5 to 500 potent hits are profiled.

10. Method of claim 1 or 2 wherein said library of compounds comprises between 5 000 and 500 000 compounds.

11. Method of claim 8 wherein said library of compounds comprises between 10 000 and 20 000 compounds.

12. Method of claim 2 wherein said active compounds are drug candidates.

13. Method of claim 2 wherein said active compounds are receptor ligands.

14. A method of High Throughput Profiling comprising the screening of a large number of compounds for their capacity to interact with one given target amongts a large number of targets.

15. A focused library comprising a plurality of compounds, wherein said compounds have structural diversity and derive from compounds having the capacity to bind selectively to a common desired target.

16. Compounds CER703950, CER704252, CER704261, CER704478, CER704889, CER709929, CER710110, CER710830, CER812221, CER833240, CER703696, and CER788867 as represented in Figure 4.
